# EUROPÄISCHE PATENTSCHRIFT

(11) **EP 3 287 232 B1**
(45) Veröffentlichungstag und Bekanntmachung des Hinweises auf die Patenterteilung: **10.11.2021**
(21) Anmeldenummer: 17191893.1
(22) Anmeldetag: 03.06.2014
(51) Int. Cl.: B23Q 11/08

(54) **EIGENSTEIFE TELESKOPISCHE SCHUTZABDECKUNG**
INHERENTLY RIGID TELESCOPIC PROTECTIVE COVER
CAPOT DE PROTECTION TÉLÉSCOPIQUE À RIGIDITÉ INTRINSÈQUE

(30) Priorität: 05.06.2013 DE 102013210407
(43) Veröffentlichungstag der Anmeldung: 28.02.2018
(62) Teilanmeldung aus: 14732101.2
(73) Patentinhaber: Arno Arnold GmbH, 63179 Obertshausen (DE)
(72) Erfinder: KREUTZER, Thomas, 63179 Obertshausen (DE); CHRISTLIEB, Thomas, 63179 Obertshausen (DE); GRIMM, Alexander, 63179 Obertshausen (DE); ROEHNERT, Maximilian, 63179 Obertshausen (DE); BOCK, Tatjana, 63179 Obertshausen (DE); WEISE, Peter, 63179 Obertshausen (DE); GAWLIK, Josef, 63179 Obertshausen (DE)
(74) Vertreter: advotec.

(56) Entgegenhaltungen:
- WO-A1-2012/041293
- DE-A1- 10 308 024
- DE-A1-102010 045 810
- JP-A- 2007 144 534
- JP-A- 2008 080 463

## Beschreibung

Die Erfindung betrifft eine Schutzabdeckung gemäß dem Oberbegriff des Patentanspruchs 1 (siehe z.B. DE 10 2010 045810 A1).

Schutzabdeckungen der eingangs genannten Art dienen beispielsweise bei Werkzeugmaschinen dazu, bewegliche Teile wie Antriebe und dergleichen gegen unbeabsichtigten Zugriff eines Benutzers der Maschine abzusichern, und zudem den Arbeitsraum der Werkzeugmaschine derart abzuschließen, dass keine Späne, flüssige Medien oder andere Teile und Partikel in einer den Benutzer gefährdenden und/oder andere Maschinenbereiche verunreinigenden Weise aus- bzw. eintreten können.

Übliche gattungsgemäße Schutzabdeckungen weisen dazu Schutzabdeckungssegmente in Form von im Wesentlichen L-förmigen Blechelementen auf, die teleskopartig gegeneinander verschiebbar sind, sich dabei gegenseitig mit einem ihrer Schenkel hintergreifen, und so eine im wesentlichen geschlossene, bewegliche und längenveränderbare Abdeckung bilden.

Da hierbei die einander übergreifenden Abdeckschenkel lediglich weitgehend lose aufeinanderliegen, ist oft entweder kein ausreichend dichter Abschluss möglich, oder es müssen konstruktiv aufwendige Ergänzungen vorgenommen werden. So werden gattungsgemäße Schutzabdeckungen üblicherweise zusätzlich mit einem Faltenbalg auf der Rückseite versehen, um einen vergleichsweise dichten Abschluss zu gewährleisten, wobei der Faltenbalg auf geeignete Weise mit den Blechelementen der Schutzabdeckung verbunden werden muss.

Auch sind Schutzabdeckungen bekannt, bei welcher der rückwärtige Faltenbalg beispielsweise an Laschen befestigt ist, die an die Blechelemente der Schutzabdeckung angeformt sind. Hierzu müssen jedoch ggf. Schlitze in den Faltenbalg gestanzt werden, welche zur Befestigung des Faltenbalgs an den Blechelementen der Schutzabdeckung von den Laschen der Blechelemente durchgriffen werden. Hierdurch können sich Schwachstellen bezüglich der Abdichtungswirkung der Schutzabdeckung bzw. des Faltenbalgs ergeben, da Medien oder Verunreinigungen die Schlitze im Faltenbalg durchdringen können.

Zur Verbesserung der Dichtigkeit bzw. des gleichmäßigen Zusammenhalts der aneinander anliegenden Blechelemente ist es beispielsweise aus der WO2012175232A1 und aus der DE102005013496B4 ferner bekannt, federnde Rückstellelemente zwischen den Blechelementen so anzuordnen, dass geeignete Rückstellkräfte bzw. entsprechende Drehmomente erzeugt und auf die Blechelemente übertragen werden. Unter anderem sollen die Rückstellkräfte bzw. Drehmomente dazu führen, dass die einander übergreifenden Abdeckschenkel der Schutzabdeckung nicht mehr nur lose aufeinander liegen, sondern mittels einer Anpresskraft aufeinander gedrückt werden.

Die hierzu zusätzlich notwendigen Rückstellelemente und deren Befestigung an den Blechelementen verursachen jedoch zusätzlichen Herstellungs- und Montageaufwand. Zudem vergrößern die Rückstellelemente und deren Befestigung an den Blechelementen das Blockmaß der Schutzabdeckung, wenn diese auf ihre minimale Länge zusammengeschoben werden soll.

All dies macht derartige Schutzabdeckungen aufwendig herzustellen und vergleichsweise teuer. Schließlich wird hierdurch auch das Gewicht der Schutzabdeckung erhöht, so dass Schutzabdeckungen größerer Länge aufgrund ihres erhöhten Gewichts nicht mehr mit der erforderlichen Geschwindigkeit bzw. Beschleunigung verfahren werden können.

Die DE 10 2010 045 810 A1 offenbart eine längenveränderliche Abdeckung mit einem Faltenelement, welches zumindest drei Kanten aufweist. Dabei ist das Faltenelement im Bereich der ersten Kante, einer Faltenspitze, einer Mehrzahl sich in Auszugsrichtung X des Abdeckelements überlappender Lamellen zugewandt. Weiter ist das Faltenelement im Bereich der zweiten Kante, der gegenüberliegenden Faltenspitze, von den Lamellen abgewandt und im Bereich der zwischen der ersten und der zweiten Kante angeordneten dritten Kante an einem Ende der Lamelle angeordnet.

Ausgehend von diesem Stand der Technik ist es somit die Aufgabe der vorliegenden Erfindung, eine Schutzabdeckung zu schaffen, die bei vergleichsweise geringem Gewicht und vergleichsweise geringen Herstellungskosten einen sicheren und dichten Abschluss voneinander zu trennender Arbeitsbereiche insbesondere an einer Werkzeugmaschine gewährleistet.

Diese Aufgabe wird durch eine Schutzabdeckung nach der Lehre des Patentanspruchs 1 gelöst. Vorteilhafte Ausgestaltungen der Erfindung sind Gegenstand der Unteransprüche.

Die Schutzabdeckung gemäß der vorliegenden Erfindung weist in zunächst bekannter Weise eine Mehrzahl von im Wesentlichen L-förmigen Schutzabdeckungssegmenten mit jeweils einem Abdeckschenkel und jeweils einem Stützschenkel auf. Diese Schutzabdeckungssegmente sind dabei so relativ zueinander angeordnet, dass sie teleskopartig gegeneinander verschiebbar sind, wobei jeweils die Abdeckschenkel benachbarter Schutzabdeckungssegmente unter Bildung einer insgesamt im Wesentlichen geschlossenen Fläche aufeinander zur Anlage gelangen. Weiter ist mindestens ein band- oder bahnförmiges flexibles Verbindungselement vorgesehen, das die Stützschenkel der Schutzabdeckungssegmente nach Art eines Faltenbalgs ziehharmonikaartig miteinander verbindet. Dabei weist das Verbindungselement mindestens eine dem Abdeckschenkel zugewandte Kante und mindestens eine dem Abdeckschenkel abgewandte Kante auf.

In zunächst ebenfalls bekannter Weise ist dabei am Stützschenkel des jeweiligen Schutzabdeckungssegments mindestens ein elastisches Rückstellelement derart angeordnet, dass es - beim teleskopischen Auszug der Schutzabdeckung - ein Moment erzeugt und auf den Stützschenkel überträgt.

Erfindungsgemäß zeichnet sich die Schutzabdeckung jedoch dadurch aus, dass das elastische Rückstellelement einstückiger Bestandteil des Verbindungselements ist. Mit anderen Worten wird die Funktion des elastischen Rückstellelements bei der Erfindung durch das Verbindungselement bzw. den Faltenbalg selbst übernommen, so dass das beim Stand der Technik als zusätzliches Bauteil erforderliche Rückstellelement sowie dessen Befestigung entfallen können. Dies wird dadurch erreicht, dass das Verbindungselement in Form eines eigensteifen Bandes bzw. einer biegesteifen Bahn ausgebildet ist, wobei Band bzw. Bahn entlang eines Höhenbereichs des Stützschenkels flächig mit dem Stützschenkel des Schutzabdeckungssegments verbunden ist und wobei der Höhenbereich näher an der dem Abdeckschenkel abgewandten Kante des Verbindungselementes als an der dem Abdeckschenkel zugewandten Kante des Verbindungselementes anordenbar ist.

Aufgrund des Einsatzes eines eigensteifen Bandes als Verbindungselement und aufgrund dessen nicht nur punktuellen oder linienförmigen, sondern eine Fläche entlang eines Höhenbereichs des Stützschenkels umfassenden Verbindung mit dem Stützschenkel können sowohl das beim Stand der Technik zusätzlich erforderliche elastische Rückstellelement, als auch dessen Befestigung am Stützschenkel, ebenso wie die beim Stand der Technik erforderliche zusätzliche Verbindung zwischen den Schutzabdeckungssegmenten und dem flexiblen Verbindungselement bzw. Faltenbalg vollständig entfallen.

Anstelle durch das (entfallene) Rückstellelement werden die gewünschten Kräfte, Momente und Anpresskräfte zwischen den Abdeckschenkeln der Schutzabdeckungssegmente erfindungsgemäß durch das als eigensteifes Band ausgebildete Verbindungselement selbst erzeugt. Hierzu wird beim teleskopischen Auszug der Schutzabdeckung vom Verbindungselement aufgrund dessen Biegesteifigkeit bzw. Eigensteifigkeit und aufgrund dessen eine Fläche umfassenden Verbindung mit dem Stützschenkel ein Moment auf das Schutzabdeckungssegment induziert, welches dieses zu verkippen versucht. Das Moment führt somit dazu, dass die Kanten der teleskopartig übereinander greifenden Abdeckschenkel auf die Flächen der jeweils benachbarten Abdeckschenkel gedrückt werden, wodurch sich insgesamt ein sicherer und dichter Abschluss der Schutzabdeckung ergibt.

Das Verbindungselement bzw. der Faltenbalg übernimmt dank der Erfindung somit nicht nur die abdichtende und zusätzlich die Funktion der (mit der Erfindung entfallenen) Federelemente zwischen den Schutzabdeckungssegmenten, sondern erzeugt zusätzlich auch noch ein Moment, welches stabilisierend auf die Schutzabdeckungssegmente wirkt und damit deren gleichmäßige Bewegung und Dichtungswirkung weiter verbessert.

Ausführung und Materialwahl für das Verbindungselement ebenso wie die Ausgestaltung der flächigen bzw. eine Fläche übergreifenden Befestigung des Verbindungselements entlang eines Höhenbereichs des Stützschenkels ist grundsätzlich beliebig, solange vom Verbindungselement einerseits die zum gleichmäßigen teleskopischen Auseinanderziehen notwendige Kraft auf die Schutzabdeckungssegmente übertragbar ist, und andererseits beim Auseinanderziehen der Schutzabdeckung durch das Verbindungselement das gewünschte Moment erzeugbar und auf das Schutzabdeckungssegment übertragbar ist.

Nach einem ersten bevorzugten Ausführungsbeispiel der Erfindung ist das Verbindungselement an beiden Flächenseiten des Stützschenkels entlang im wesentlichen gleichgroßer Höhenbereiche am Stützschenkel befestigt. Diese Ausführungsform ermöglicht eine besonders einfache Herstellung der Verbindung zwischen dem Verbindungselement und den Schutzabdeckungssegmenten. Gleichzeitig wird mit dieser Ausführungsform die gewünschte Stabilisierungswirkung für die Schutzabdeckungssegmente erzeugt, indem die flächige, momentenübertragende Befestigung des Verbindungselements an den Schutzabdeckungssegmenten, zusammen mit der Eigensteifigkeit des Materials des Verbindungselements dazu führt, dass beim teleskopischen Auszug der Schutzabdeckung sowie aufgrund der geometrisch bedingten Verkippung der ineinander greifenden Schutzabdeckungssegmente jeweils Drehmomente im Verbindungselement induziert und auf die Schutzabdeckungssegmente übertragen werden, die zum gewünschten Anpressdruck benachbarter Schutzabdeckungssegmente aufeinander führen.

Eine weitere Ausführungsform der Erfindung sieht vor, dass der Höhenbereich der Befestigung des Verbindungselements an der dem Abdeckschenkel zugewandten Flächenseite des Stützschenkels größer ist als der Höhenbereich der Befestigung des Verbindungselements an der dem Abdeckschenkel abgewandten Flächenseite des Stützschenkels. Diese Ausführungsform schließt insbesondere weitere, nachstehend noch beschriebene Ausführungsformen ein, bei denen der Höhenbereich der Befestigung des Verbindungselements an der dem Abdeckschenkel abgewandten Seite des Stützschenkels gleich null ist, bzw. bei denen das Verbindungselement überhaupt nicht an der letztgenannten Seite des Stützschenkels, sondern nur an der dem Abdeckschenkel zugewandten Seite des Stützschenkels befestigt ist.

Diese Ausführungsform hat insbesondere den Vorteil, dass beim teleskopischen Auszug der Schutzabdeckung - aufgrund der mit dieser Ausführungsform erzielten stark asymmetrischen Verformung des Verbindungselements beim Auszug - wesentlich größere Momente durch die Schutzabdeckung erzeugt und auf die Schutzabdeckungssegmente übertragen werden, wodurch sich eine erhöhte Dichtigkeit und noch bessere gegenseitige Abstreifwirkung zwischen den aneinander aufliegenden Abdeckschenkeln der Schutzabdeckung ergibt.

Auf welche Weise die unterschiedliche Größe des Höhenbereichs der Befestigung des Verbindungselements an den beiden Flächenseiten des Stützschenkels, bzw. ggf. eine Befestigung des Verbindungselements an nur einer Flächenseite des Stützschenkels konstruktiv erreicht wird, ist zunächst einmal beliebig, solange von der Befestigung die erforderlichen Kräfte und Momente übertragen werden können.

Gemäß weiterer Ausführungsformen jedoch ist es vorgesehen, dass der Stützschenkel innerhalb des Höhenbereichs der Befestigung des Verbindungselements am Stützschenkel zumindest einen, vorzugsweise jedoch eine Mehrzahl bevorzugt kreis- oder ellipsenförmiger Durchbrüche aufweist, die zur Befestigung des Verbindungselements am Stützschenkel herangezogen werden können. Die Befestigung des Verbindungselements an bzw. in Durchbrüchen der Stützschenkel ist zunächst einmal konstruktiv besonders einfach und damit kostengünstig realisierbar.

Mit diesem Hintergrund sieht eine weitere Ausführungsform der Erfindung vor, dass zwei Flanken des Verbindungselements im Bereich des Stützschenkels den zumindest einen am Stützschenkel angeordneten Durchbruch durchgreifen und innerhalb des Durchbruchs miteinander verbunden sind. Die Verbindung der beiden Flanken des Verbindungselements innerhalb des Durchbruchs erfolgt dabei bevorzugt stoffschlüssig, insbesondere durch Schweißen, beispielsweise Ultraschallschweißen.

Auf diese Weise ergibt sich eine einfache und kostengünstige Befestigung des Verbindungselements am Stützschenkel, ohne dass hierbei zusätzliche Befestigungsmaterialien oder Befestigungselemente erforderlich sind.

Gemäß einer weiteren Ausführungsform ist im Bereich des zumindest einen Durchbruchs am Stützschenkel ein beide Flächenseiten des Stützschenkels umgreifender Befestigungsstreifen angeordnet, dessen beide Flanken den zumindest einen Durchbruch durchgreifen und innerhalb des zumindest einen Durchbruchs, wie vorstehend, wieder bevorzugt stoffschlüssig miteinander verbunden sind. Dabei ist zudem das Verbindungselement entlang eines Höhenbereichs des Stützschenkels und des Befestigungsstreifens flächig mit dem Befestigungsstreifen verbunden, wieder vorzugsweise verschweißt. Bei dieser Ausführungsform ergibt sich eine besonders robuste Befestigung des Verbindungselements am Stützschenkel und eine gute Kraft- sowie Momentenübertragung zwischen Verbindungselement und Stützschenkel, aufgrund der hier möglichen, großflächigen Verschweißung zwischen Verbindungselement und Befestigungsstreifen, welcher Kräfte und erzeugte Momente somit ebenfalls großflächig in den Stützschenkel einleitet.

Eine weitere Ausführungsform sieht vor, dass im Bereich des zumindest einen Durchbruchs am Stützschenkel auf der dem Abdeckschenkel abgewandten Flächenseite des Stützschenkels ein Befestigungsstreifen angeordnet ist. Dabei durchgreifen der Befestigungsstreifen und die dem Abdeckschenkel zugewandte Flanke des Verbindungselements den zumindest einen Durchbruch und sind innerhalb des zumindest einen Durchbruchs bevorzugt stoffschlüssig miteinander verbunden.

Bei dieser Ausführungsform ergibt sich eine einfache Befestigung des Verbindungselements am Stützschenkel durch (minimal) nur einen Befestigungsstreifen pro Schutzabdeckungssegment, der jeweils alle an einem Schutzabdeckungssegment angeordneten Durchbrüche abdeckt. Ferner ergibt sich eine Verringerung der Dicke bzw. des Blockmaßes der Schutzabdeckung im zusammengeschobenen Zustand aufgrund des hier nur einseitig am Stützschenkel angeordneten Befestigungsstreifens.

Gemäß einer weiteren Ausführungsform ist vorgesehen, dass auf der dem Abdeckschenkel abgewandten Flächenseite des Stützschenkels jeweils ein Befestigungselement, vorzugsweise Niet, in dem zumindest einen Durchbruch des Stützschenkels angeordnet ist. Dabei durchgreifen das Befestigungselement und die dem Abdeckschenkel zugewandte Flanke des Verbindungselements gemeinsam den zumindest einen Durchbruch, und sind innerhalb des Durchbruchs, wie bei den vorstehenden Ausführungsformen vorzugsweise stoffschlüssig, miteinander verbunden.

Eine weitere Ausführungsform für die Befestigung des Verbindungselements am Stützschenkel des Schutzabdeckungssegments sieht vor, dass das Verbindungselement an der dem Abdeckschenkel zugewandten Flächenseite des Stützschenkels mittels eines einen Höhenbereich abdeckenden Klebebands mit dem Stützschenkel verbunden ist.

Diese Ausführungsform hat den Vorteil einer einfachen, platzsparenden und großflächigen Befestigung des Verbindungselements am Stützschenkel. Bei dem Klebeband kann es sich zum Beispiel um beidseitig klebendes Klebeband handeln, oder beispielsweise um ein einseitig beschichtetes Hotmeltfilmband, das mit der Hotmelt-beschichteten Seite auf den Stützschenkel geklebt, und auf der unbeschichteten Rückseite mit dem Verbindungselement verbunden, vorzugsweise verschweißt wird.

Das Material des Verbindungselements bzw. Faltenbalgs der erfindungsgemäßen Schutzabdeckung ist zunächst beliebig, solange die zur Erzeugung und Übertragung eines Drehmoments auf die Schutzabdeckungssegmente erforderlichen Biegemomente (mittels Durchbiegung des band- oder bahnförmigen Verbindungselements) erzeugt werden können, mit anderen Worten, solange es sich um ein band- oder bahnförmiges Material handelt, welches eine gewisse Eigen- bzw. Biegesteifigkeit aufweist, also nicht (wie beispielsweise rein textiles Material oder dünne Folien) im Wesentlichen biegeschlaff ist.

Eine bevorzugte Ausführungsform der Erfindung jedoch sieht vor, dass das Verbindungselement aus einem eigensteifen polymeren Bahnmaterial, vorzugsweise aus Polypropylen besteht. Derartige polymere Materialien wie z.B. gewisse Thermoplaste sind kostengünstig, und sen - insbesondere im Fall der Verwendung von Polypropylen - zudem eine hohe Beständigkeit gegen wässrige Medien, Säuren, Laugen, Öle und andere Chemikalien auf, was für den vorgesehenen Einsatzzweck als Werkzeugmaschinen-Schutzabdeckung von zusätzlichem Vorteil ist. Die Materialstärke des Verbindungselements liegt dabei vorzugsweise zwischen 0,3 und 1,5 mm, besonders bevorzugt zwischen 0,5 und 1,0 mm.

Diese Dickenabmessungen haben sich im Hinblick auf Betriebssicherheit einerseits und Erzeugung der erforderlichen Biegemomente im Bahnmaterial des Verbindungselements bzw. Faltenbalgs andererseits bewährt.

Im Folgenden wird die Erfindung anhand lediglich Ausführungsbeispiele darstellender Zeichnungen näher erläutert.

Dabei zeigt
- **Fig. 1**: in schematischer Darstellung eine Schutzabdeckung gemäß einer Ausführungsform der vorliegenden Erfindung in einer Seitenansicht;
- **Fig. 2**: in einer **Fig. 1** entsprechenden Darstellung ein Segment einer Schutzabdeckung gemäß einer weiteren Ausführungsform;
- **Fig. 3**: in einer **Fig. 2** entsprechenden Darstellung und Ansicht ein Segment einer Schutzabdeckung mit beidseitigem Befestigungs streifen;
- **Fig. 4**: in einer **Fig. 2** und 3 entsprechenden Darstellung und Ansicht ein Segment einer Schutzabdeckung mit einseitigem Befestigungsstreifen;
- **Fig. 5**: in einer **Fig. 2** bis **4** entsprechenden Darstellung und Ansicht ein Segment einer Schutzabdeckung mit Nietbefestigung; und
- **Fig. 6**: in einer **Fig. 2** bis **4** entsprechenden Darstellung und Ansicht ein Segment einer Schutzabdeckung mit Klebebandbefestigung.

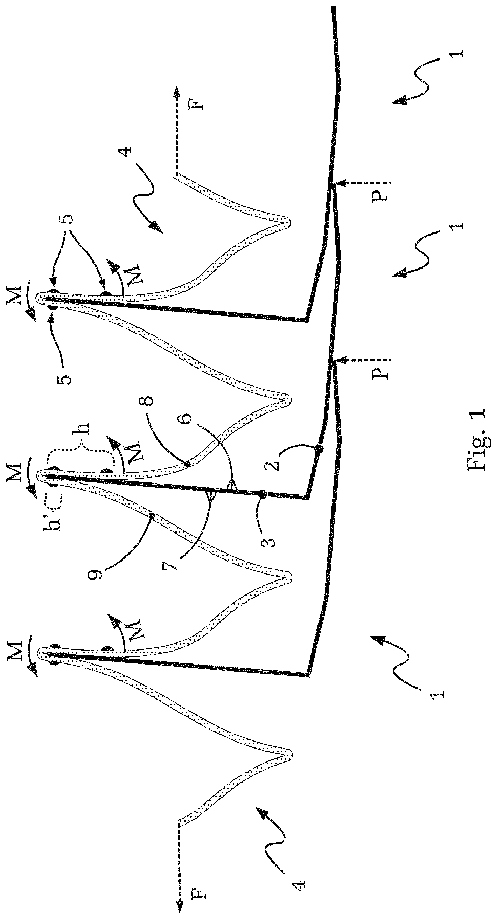

**Fig. 1** zeigt in einer schematischen Darstellung einen Ausschnitt aus einer Schutzabdeckung gemäß einer Ausführungsform der vorliegenden Erfindung. Man erkennt zunächst drei Schutzabdeckungssegmente 1, die zeichnungsbezogen in horizontaler Richtung teleskopartig gegeneinander und ineinander verschiebbar sind. Die Schutzabdeckungssegmente 1 weisen jeweils einen Abdeckschenkel 2 und einen Stützschenkel 3 auf.

Die Schutzabdeckungssegmente 1 sind in für sich genommen zunächst bekannter Weise mittels eines bahnförmigen Verbindungselements bzw. Faltenbalgs 4 miteinander verbunden.

Das Material des Verbindungselements bzw. Faltenbalgs 4 ist erfindungsgemäß jedoch eigensteif bzw. biegesteif ausgebildet. Zudem ist die Befestigung des Faltenbalgs 4 an den Stützschenkeln 3 so ausgebildet, dass sich Höhenbereiche h und h' ergeben, entlang bzw. innerhalb derer der Faltenbalg 4 mit dem jeweiligen Stützschenkel 3 flächig verbunden ist.

Die in Fig. 1 angedeuteten Niete 5 dienen lediglich der schematischen Visualisierung einer Verbindung zwischen Faltenbalg 4 und Stützschenkel 3, und stellen damit eine zwar mögliche, jedoch nicht eine besonders bevorzugte Ausführungsform dieser Verbindung dar.

Man erkennt in Fig. 1, dass der Höhenbereich h auf der zeichnungsbezogen rechten, also dem Abdeckschenkel 2 jeweils zugewandten Flächenseite 6 des Stützschenkels 3, wesentlich größer ist als der Höhenbereich h' auf der zeichnungsbezogen linken, also dem Abdeckschenkel 2 jeweils abgewandten Flächenseite 6 des Stützschenkels 3. Auf diese Weise ergeben sich bezüglich der zeichnungsbezogen jeweils rechtsseitigen Flanken 8 des Faltenbalgs 4 größere Hebelarme und zudem eine jeweils stärkere Durchbiegung der zeichnungsbezogen rechtsseitigen Flanken 8 des Faltenbalgs 4, im Vergleich zu den zeichnungsbezogen linksseitigen Flanken 9. Bei den letztgenannten fällt sowohl die Durchbiegung als auch der Hebelarm des Angriffs am Stützschenkel 3 kleiner aus.

Aufgrund der stärkeren Durchbiegung der zeichnungsbezogen rechtsseitigen Flanken 8 des eigensteifen Faltenbalgs 4, sowie aufgrund des dort größeren Höhenbereichs h der Verbindung zwischen Faltenbalg 4 und Stützschenkel 3 und des damit dort größeren Hebelarms, ergibt sich anhand der Auszugskraft F in den Flanken 8, 9 des Faltenbalgs 4 effektiv ein Drehmoment M, welches über die Verbindung 5 zwischen Faltenbalg 4 und Stützschenkel 3 auf das jeweilige Schutzabdeckungssegment 1 übertragen wird. Dies bedeutet mit anderen Worten, dass der Faltenbalg 4 versucht, das jeweilige Schutzabdeckungssegment 1 zeichnungsbezogen nach links zu verdrehen, und zwar umso stärker, je weiter der Faltenbalg 4 mittels der Kraft F auseinandergezogen wird.

Hierdurch entsteht - zusätzlich zu einer sehr gleichmäßigen Verteilung der Auszugskraft F auf die einzelnen Lamellen bzw. Schutzabdeckungssegmente 1 der Schutzabdeckung - im Bereich der linienförmigen gegenseitigen Anlage der Abdeckschenkel 2 eine Anpresskraft P, welche die Kanten der Abdeckschenkel 2 auf die Flächen der jeweils benachbarten Abdeckschenkel 2 drückt. Insgesamt ergibt sich hierdurch in der gewünschten Weise eine verbesserte Dichtigkeit, Stabilität und Betriebssicherheit der erfindungsgemäßen Schutzabdeckung, ohne dass hierzu eigens zusätzlich vorzusehende und zu befestigende Rückstellfederelemente oder dergleichen zwischen den Stützschenkeln 3 der Schutzabdeckung erforderlich sind, wie dies im Stand der Technik der Fall ist.

Neben dem Wegfall der Rückstellfederelemente und deren Befestigung an den Schutzabdeckungssegmenten entfällt auch das zusätzliche Dickenmaß, das durch diese Elemente jedem Schutzabdeckungssegment hinzugefügt wird. Dies führt zu einer erheblichen Verkleinerung des Blockmaßes der Schutzabdeckung im zusammengeschobenen Zustand, wie auch zu einer Verringerung der Masse der Schutzabdeckung, was bei hohen Verfahrgeschwindigkeiten bzw. -beschleunigungen von maßgeblicher Bedeutung ist.

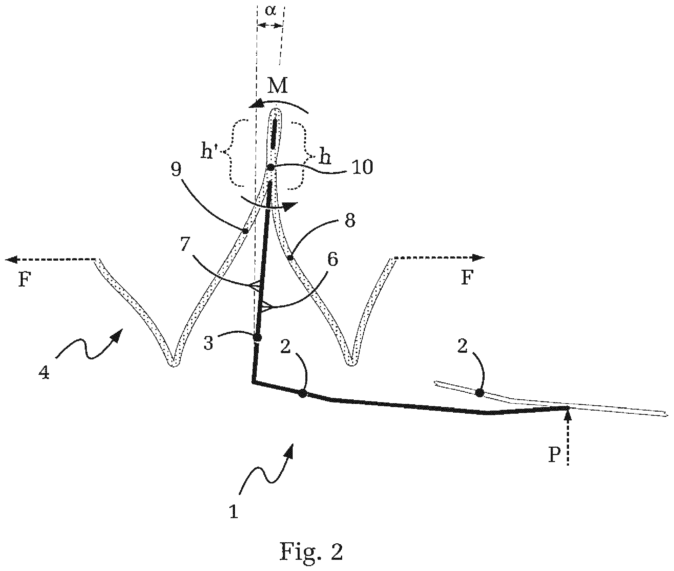

Fig. 2 zeigt ein Segment einer Schutzabdeckung gemäß einer weiteren Ausführungsform der Erfindung. Bei dieser Ausführungsform sind im Stützschenkel 3 innerhalb des Höhenbereichs h bzw. h' der Befestigung des Faltenbalgs 4 entlang der Querrichtung der Schutzabdeckung (senkrecht zur Zeichnungsebene der Fig. 2) eine Anzahl bevorzugt kreis- oder ellipsenförmiger Durchbrüche 10 zum Zweck der Befestigung des Faltenbalgs 4 am Stützschenkel 3 angeordnet.

Die beiden Flanken 8 und 9 des Faltenbalgs 4 sind dabei im Bereich der Durchbrüche 10 miteinander verbunden, beispielsweise ultraschallverschweißt, so dass sich eine formschlüssige Verbindung zwischen Faltenbalg 4 und Stützschenkel 3 entlang des Höhenbereichs h bzw. h' ergibt. Bei dieser Ausführungsform sind die zeichnungsbezogen linken und rechten Höhenbereiche h und h' etwa gleich groß. Dennoch wird vom Faltenbalg 4 auch bei dieser Ausführungsform - aufgrund der im Betrieb des Schutzabdeckung auftretenden Verkippung der Schutzabdeckungssegmente 1 - ein zeichnungsbezogen linksdrehendes Drehmoment M erzeugt, welches wieder wie gewünscht dazu führt, dass die Kanten der Abdeckschenkel 2 der Schutzabdeckungssegmente 1 mit der Kraft P auf den jeweils benachbarten Abdeckschenkel 2 gepresst werden.

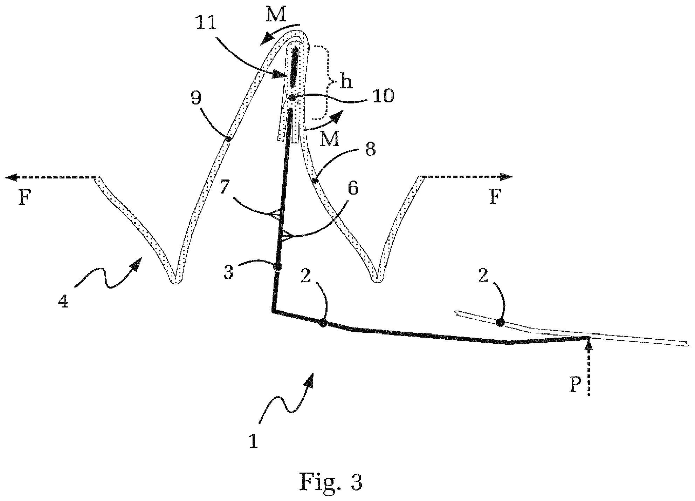

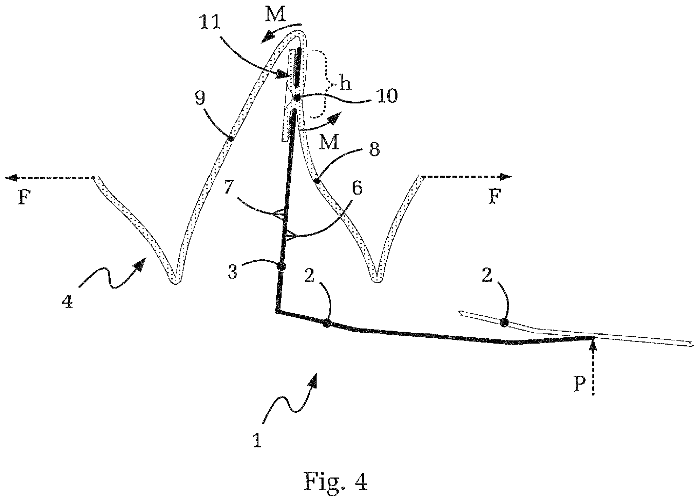

Fig. 3 und 4 zeigen weitere Ausführungsformen, bei denen die Verbindung zwischen Faltenbalg 4 und Stützschenkel 3 jeweils mittels eines Befestigungsstreifens 11 erfolgt, welcher innerhalb des Höhenbereichs h am Stützschenkel 3 angeordnet und seinerseits mit dem Faltenbalg 4 verbunden ist. Auf diese Weise ergibt sich im Höhenbereich h jeweils eine Verbindung zwischen Faltenbalg 4 und Stützschenkel 3 bzw. Schutzabdeckungssegment 1, welche lediglich die zeichnungsbezogen rechtsseitige Flanke 8 des Faltenbalgs 4 umfasst. Hierdurch wird, wie bereits im Hinblick auf Fig. 1 beschrieben, aufgrund der unterschiedlichen Durchbiegung der beiden Flanken 8, 9 des Faltenbalgs 4 ein entsprechendes effektives Moment M erzeugt, welches die Schutzabdeckungssegmente 1 in der gewünschten Weise gegen den Uhrzeigersinn zu verdrehen versucht und damit die Anpresskraft P erzeugt.

Bei der Ausführungsform gemäß Fig. 3 ist der Befestigungsstreifen 11 über die zeichnungsbezogen obere Kante des Stützschenkels 3 geklappt und umgreift diesen somit im Bereich seiner beiden Flächenseiten 6, 7, wobei die beiden Seiten des Befestigungsstreifens 11 - wieder im Bereich der Durchbrüche 10 des Stützschenkels 3 - miteinander verbunden, vorzugsweise verschweißt sind. Mit dem Befestigungsstreifen 11 wiederum ist sodann die rechte Flanke 8 des Faltenbalgs 4 verbunden, vorzugsweise ebenfalls verschweißt.

Bei der Ausführungsform gemäß Fig. 4 ist der Befestigungsstreifen 11 lediglich einseitig an der zeichnungsbezogen linken Flächenseite 7 des Stützschenkels 3 angeordnet, entweder lose oder angeklebt, und im Bereich der Durchbrüche 10 unmittelbar mit der zeichnungsbezogen rechten Flanke 8 des Faltenbalgs 4 verbunden. Diese Ausführungsform ermöglicht insbesondere ein geringeres Blockmaß der Schutzabdeckung im komplett zusammengeschobenen Zustand.

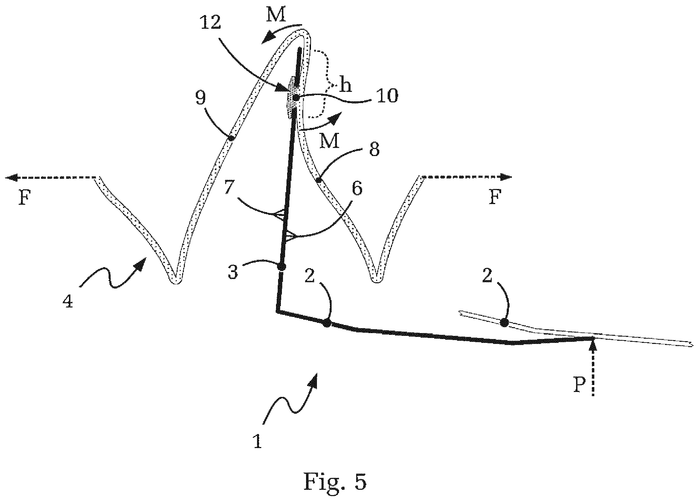

Fig. 5 zeigt eine weitere Ausführungsform, die sich von der Ausführungsform gemäß Fig. 4 dadurch unterscheidet, dass anstelle des Befestigungsstreifens 11 - zur Verbindung zwischen der zeichnungsbezogen rechtsseitigen Flanke 8 des Faltenbalgs 4 mit dem Stützschenkel 3 - Niete 12 eingesetzt sind, welche in den Durchbrüchen 10 mit der Flanke 8 des Faltenbalgs 4 stoffschlüssig verbunden, vorzugsweise verschweißt sind. Diese Ausführungsform ermöglicht eine besonders material-, gewichts- und platzsparende Befestigung des Faltenbalgs 4 am Stützschenkel 3, insbesondere dann, wenn Durchbrüche 10 und Niete 12 bei benachbarten Schutzabdeckungssegmenten 1 jeweils versetzt angeordnet werden.

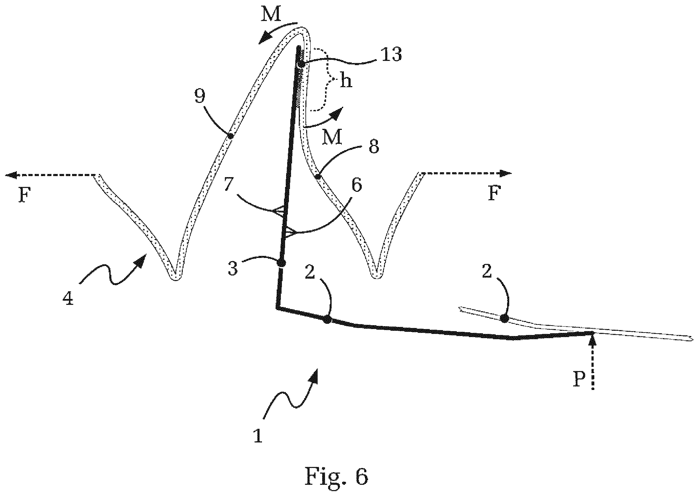

Bei der Ausführungsform gemäß Fig. 6 erfolgt die Befestigung der rechtsseitigen Flanke 8 des Faltenbalgs 4 mittels eines den Höhenbereich h abdeckenden Klebebands 13, beispielsweise mittels eines doppelseitigen Klebebands oder mittels eines Hotmeltfilmbands, welches auf der zeichnungsbezogen linken Oberfläche beispielsweise eine Hotmeltschicht aufweist und auf der zeichnungsbezogen rechtsseitigen Rückseite mit der Flanke 8 des Faltenbalgs 4 beispielsweise verschweißt wird.

## Patentansprüche

1. Schutzabdeckung mit einer Mehrzahl von im Wesentlichen L-förmigen Schutzabdeckungssegmenten (1) mit jeweils einem Abdeckschenkel (2) und jeweils einem Stützschenkel (3), die so relativ zueinander angeordnet sind, dass sie teleskopartig gegeneinander verschiebbar sind, wobei jeweils die Abdeckschenkel (2) benachbarter Schutzabdeckungssegmente (1) unter Bildung einer insgesamt im Wesentlichen geschlossenen Fläche aufeinander zur Anlage gelangen, und mit mindestens einem flexiblen, band- oder bahnförmig ausgebildeten Verbindungselement (4), das die Stützschenkel (3) der Schutzabdeckungssegmente (1) nach Art eines Faltenbalgs (4) ziehharmonikaartig miteinander verbindet,
wobei das Verbindungselement (4) mindestens eine dem Abdeckschenkel (2) zugewandte Kante und mindestens eine dem Abdeckschenkel (2) abgewandte Kante aufweist,
wobei am Stützschenkel (3) jeweils mindestens ein elastisches Rückstellelement derart angreift, dass beim teleskopischen Auszug der Schutzabdeckung ein Moment (M) erzeugt und auf den Stützschenkel (3) übertragen wird,
**dadurch gekennzeichnet,**
**dass** das elastische Rückstellelement einstückiger Bestandteil des Verbindungselements (4) ist, indem das Verbindungselement (4) biegesteif ausgebildet ist und entlang eines Höhenbereichs (h) des Stützschenkels (3) flächig mit dem Stützschenkel (3) verbunden ist, wobei der Höhenbereich (h) näher an der dem Abdeckschenkel (2) abgewandten Kante des Verbindungselementes (4) als an der dem Abdeckschenkel (2) zugewandten Kante des Verbindungselementes (4) angeordnet ist.

2. Schutzabdeckung nach Anspruch 1,
**dadurch gekennzeichnet,**
**dass** das Verbindungselement (4) an beiden Flächenseiten (6, 7) entlang im Wesentlichen gleich großer Höhenbereiche (h) am Stützschenkel (3) befestigt ist.

3. Schutzabdeckung nach Anspruch 1,
**dadurch gekennzeichnet,**
**dass** der Höhenbereich (h) der Befestigung des Verbindungselements (4) an der dem Abdeckschenkel (2) zugewandten Flächenseite (6) des Stützschenkels (3) größer ist als der Höhenbereich (h') der Befestigung des Verbindungselements (4) an der dem Abdeckschenkel (2) abgewandten Flächenseite (7) des Stützschenkels (3).

4. Schutzabdeckung nach einem der Ansprüche 1 bis 3,
**dadurch gekennzeichnet,**
**dass** der Stützschenkel (3) innerhalb des Höhenbereichs (h) der Befestigung des Verbindungselements (4) zumindest einen Durchbruch (10) zur Befestigung des Verbindungselements (4) am Stützschenkel (3) aufweist.

5. Schutzabdeckung nach Anspruch 4,
**dadurch gekennzeichnet,**
**dass** zwei Flanken (8, 9) des Verbindungselements (4) den zumindest einen am Stützschenkel (3) angeordneten Durchbruch (10) durchgreifen und innerhalb des zumindest einen Durchbruchs (10) miteinander verbunden sind.

6. Schutzabdeckung nach Anspruch 4,
**dadurch gekennzeichnet,**
**dass** im Bereich des zumindest einen am Stützschenkel (3) angeordneten Durchbruchs (10) ein beide Flächenseiten (6, 7) des Stützschenkels (3) umgreifender Befestigungsstreifen (11) angeordnet ist, dessen beide Flanken den zumindest einen Durchbruch (10) durchgreifen und innerhalb des zumindest einen Durchbruchs (10) miteinander verbunden sind, wobei das Verbindungselement (4) entlang eines Höhenbereichs (h) des Stützschenkels (3) und des Befestigungsstreifens (11) flächig mit dem Befestigungsstreifen (11) verbunden ist.

7. Schutzabdeckung nach Anspruch 4,
**dadurch gekennzeichnet,**
**dass** im Bereich des am Stützschenkel (3) angeordneten zumindest einen Durchbruchs (10) auf der dem Abdeckschenkel (2) abgewandten Flächenseite des Stützschenkels (3) ein Befestigungsstreifen (11) angeordnet ist, wobei der Befestigungsstreifen (11) und die dem Abdeckschenkel (2) zugewandte Flanke (8) des Verbindungselements (4) den zumindest einen Durchbruch (10) durchgreifen und innerhalb des zumindest einen Durchbruchs (10) miteinander verbunden sind.

8. Schutzabdeckung nach Anspruch 4,
**dadurch gekennzeichnet,**
**dass** auf der dem Abdeckschenkel (2) abgewandten Flächenseite (7) des Stützschenkels (3) jeweils ein Befestigungselement (12) in dem zumindest einen Durchbruch (10) des Stützschenkels (3) angeordnet ist, wobei das Befestigungselement (12) und die dem Abdeckschenkel (2) zugewandte Flanke (8) des Verbindungselements (4) den zumindest einen Durchbruch (10) durchgreifen und innerhalb des zumindest einen Durchbruchs (10) miteinander verbunden sind.

9. Schutzabdeckung nach Anspruch 3,
**dadurch gekennzeichnet,**
**dass** das Verbindungselement (4) an der dem Abdeckschenkel (2) zugewandten Flächenseite (6) des Stützschenkels (3) mittels eines den Höhenbereich (h) abdeckenden Klebebands (13) mit dem Stützschenkel (3) verbunden ist.

10. Schutzabdeckung nach einem der Ansprüche 1 bis 9,
**dadurch gekennzeichnet,**
**dass** das Verbindungselement (4) aus einem eigensteifen polymeren Bahnmaterial besteht.

11. Schutzabdeckung nach einem der Ansprüche 1 bis 10,
**dadurch gekennzeichnet,**
**dass** die Materialstärke des Verbindungselements (4) zwischen 0,3 und 1,5 mm, vorzugsweise zwischen 0,5 und 1,0 mm, beträgt.

## Claims

1. A protective cover comprising a plurality of essentially L-shaped protective cover segments (1), which each have a covering leg (2) and a supporting leg (3), which are arranged in relation to one another in such a manner that they can be moved telescopically against one another, the covering legs (2) of adjacent protective cover segments (1) respectively coming to rest on each other, thus forming an essentially completely closed surface, and comprising at least one flexible belt-shaped or web-shaped connecting element (4), which connects the supporting legs (3) of the protective cover segments (1) like an accordion in the manner of a bellows (4), wherein the connecting element (4) comprises at least one edge facing toward the covering leg (2) and at least one edge facing away from the covering leg (2),
wherein at least one elastic restoring element acts on the respective supporting leg (3) in such a manner that a torque (M) is generated and transferred to the supporting leg (3) when the protective cover is telescopically extended,
**characterised in that**
the elastic restoring element is an integral component of the connecting element (4) **in that** the connecting element (4) has flexural rigidity and is connected to the supporting leg (3) in a planar manner along a height portion (h) of the supporting leg (3) wherein the height portion (h) is arranged closer to the edge of the connecting element (4) that faces away from the covering leg (2) than to the edge of the connecting element (4) that faces toward the covering leg (2).

2. The protective cover according to claim 1,
**characterised in that**
the connecting element (4) is attached to the supporting leg (3) at both planar sides (6, 7) along essentially equally large height portions (h).

3. The protective cover according to claim 1,
**characterised in that**
the height portion (h) for attachment of the connecting element (4) to the planar side (6) of the supporting leg (3) that faces toward the covering leg (2) is larger than the height portion (h') for attachment of the connecting element (4) to the planar side (7) of the supporting leg (3) that faces away from the covering leg (2).

4. The protective cover according to any one of claims 1 to 3, **characterised in that**
the supporting leg (3) has at least one opening (10) within the height portion (h) for attachment of the connecting element (4), the opening (10) serving for attaching the connecting element (4) to the supporting leg (3).

5. The protective cover according to claim 4,
**characterised in that**
two flanks (8, 9) of the connecting element (4) penetrate the at least one opening (10) formed in the supporting leg (3) and are connected to each other within the at least one opening (10).

6. The protective cover according to claim 4,
**characterised in that**
in the area of the at least one opening (10) formed in the supporting leg (3), an attachment strip (11) is arranged, which surrounds the two planar sides (6, 7) of the supporting leg (3) and whose two flanks penetrate the at least one opening (10) and are connected to each other within the at least one opening (10), the connecting element (4) being connected to the attachment strip (11) in a planar manner along a height portion (h) of the supporting leg (3) and of the attachment strip (11).

7. The protective cover according to claim 4,
**characterised in that**
in the area of the at least one opening (10) formed in the supporting leg (3), an attachment strip (11) is arranged on the planar side of the supporting leg (3) that faces away from the covering leg (2), the attachment strip (11) and the flank (8) of the connecting element (4) that faces toward the covering leg (2) penetrating the at least one opening (10) and being connected to each other within the at least one opening (10).

8. The protective cover according to claim 4,
**characterised in that**
on the planar side (7) of the supporting leg (3) that faces away from the covering leg (2), an attachment element (12) is respectively arranged in the at least one opening (10) of the supporting leg (3), the attachment element (12) and the flank (8) of the connecting element (4) that faces toward the covering leg (2) penetrating the at least one opening (10) and being connected to each other within the at least one opening (10).

9. The protective cover according to claim 3,
**characterised in that**
on the planar side (6) of the supporting leg (3) that faces toward the covering leg (2), the connecting element (4) is connected to the supporting leg (3) by means of an adhesive tape (13) covering the height portion (h).

10. The protective cover according to any one of claims 1 to 9, **characterised in that**
the connecting element (4) is made of an inherently rigid polymer web material.

11. The protective cover according to any one of claims 1 to 10, **characterised in that**
the material thickness of the connecting element (4) ranges from 0.3 to 1.5 mm, preferably from 0.5 to 1.0 mm.

## Revendications

1. Couverture de protection comprenant une pluralité de segments de couverture de protection (1) essentiellement en forme de L, dont chacun a une branche de couverture (2) et une branche de support (3) et qui sont disposées l'une par rapport à l'autre de telle manière qu'elles puissent être déplacées télescopiquement l'une contre l'autre, les branches de couverture (2) de segments de couverture de protection (1) adjacents venant en appui respectivement l'une sur l'autre en formant une surface essentiellement fermée dans son ensemble, et ayant au moins un élément de liaison (4) flexible en forme de ruban ou de bande, ledit élément de liaison (4) reliant les branches de support (3) des segments de couverture de protection (1) à la manière d'un soufflet (4) sous forme d'accordéon,
l'élément de liaison (4) comprenant au moins un bord orienté vers la branche de couverture (2) et au moins un bord détourné de la branche de couverture (2),
au moins un élément de rappel élastique à chaque fois s'engageant à la branche de support (3) de telle manière qu'un moment (M) soit produit et transmis sur la branche de support (3) quand la couverture de protection est étendue télescopiquement,
**caractérisée en ce que**
l'élément de rappel élastique est un composant d'un seul tenant de l'élément de liaison (4), l'élément de liaison (4) étant formé rigide en flexion et étant relié à plat à la branche de support (3) le long d'une zone de hauteur (h) de la branche de support (3),
la zone de hauteur (h) étant disposée plus proche du bord de l'élément de liaison (4) détourné de la branche de couverture (2) que du bord de l'élément de liaison (4) orienté vers la branche de couverture (2).

2. Couverture de protection selon la revendication 1,
**caractérisée en ce que**
l'élément de liaison (4) est fixé à la branche de support (3) sur les deux côtés de surface (6, 7) le long de zones de hauteur (h) ayant essentiellement les mêmes dimensions.

3. Couverture de protection selon la revendication 1,
**caractérisée en ce que**
la zone de hauteur (h) pour fixer l'élément de liaison (4) sur le côté de surface (6) de la branche de support (3) orienté vers la branche de couverture (2) est plus grande que la zone de hauteur (h') pour fixer l'élément de liaison (4) sur le côté de surface (7) de la branche de support (3) détourné de la branche de couverture (2).

4. Couverture de protection selon l'une quelconque des revendications 1 à 3,
**caractérisée en ce que**
la branche de support (3) a au moins un percement (10) pour fixer l'élément de liaison (4) à la branche de support (3) dans la zone de hauteur (h) pour la fixation de l'élément de liaison (4).

5. Couverture de protection selon la revendication 4,
**caractérisée** en que
deux flancs (8, 9) de l'élément de liaison (4) s'engagent à travers ledit au moins un percement (10) disposé à la branche de support (3) et sont reliés entre eux dans ledit au moins un percement (10).

6. Couverture de protection selon la revendication 4,
**caractérisée en ce qu'**
une bande de fixation (11) entourant les deux côtés de surface (6, 7) de la branche de support (3) est disposée dans la région dudit au moins un percement (10) disposé à la branche de support (3), les deux flancs de ladite bande de fixation (11) s'engageant à travers ledit au moins un percement (10) et étant reliés entre eux dans ledit au moins un percement (10), l'élément de liaison (4) étant relié, le long d'une zone de hauteur (h) de la branche de support (3) et de la bande de fixation (11), à plat avec ladite bande de fixation (11).

7. Couverture de protection selon la revendication 4,
**caractérisée en ce qu'**
une bande de fixation (11) est disposée sur le côté de surface de la branche de support (3) détourné de la branche de couverture (2) dans la région dudit au moins un percement (10) disposé à la branche de support (3), ladite bande de fixation (11) et le flanc (8) de l'élément de liaison (4) orienté vers la branche de couverture (2) s'engageant à travers ledit au moins un percement (10) et étant reliés entre eux dans ledit au moins un percement (10).

8. Couverture de protection selon la revendication 4,
**caractérisée en ce qu'**
un élément de fixation (12) à chaque fois est disposé sur le côté de surface (7) de la branche de support (3) détourné de la branche de couverture (2) dans ledit au moins un percement (10) de la branche de support (3), ledit élément de fixation (12) et le flanc (8) de l'élément de liaison (4) orienté vers la branche de couverture (2) s'engageant à travers ledit au moins un percement (10) et étant reliés entre eux dans ledit au moins un percement (10).

9. Couverture de protection selon la revendication 3,
**caractérisée en ce que**
l'élément de liaison (4) est relié à la branche de support (3) sur le côté de surface (6) de la branche de support (3) orienté vers la branche de couverture (2) au moyen d'une bande adhésive (13) couvrant la zone de hauteur (h).

10. Couverture de protection selon l'une quelconque des revendications 1 à 9,
**caractérisée en ce que**
l'élément de liaison (4) est composé d'un matériau de bande polymère à rigidité intrinsèque.

11. Couverture de protection selon l'une quelconque des revendications 1 à 10,
**caractérisée en ce que**
l'épaisseur de matériau de l'élément de liaison (4) est compris entre 0,3 et 1,5 mm, préférentiellement entre 0,5 et 1,0 mm.
